# EUROPEAN PATENT APPLICATION

(11) **EP 0 736 248 A1**
(43) Date of publication of application: **09.10.1996**
(21) Application number: 96104878.2
(22) Date of filing: 27.03.1996
(51) Int. Cl.: A01M 1/20

(54) **Apparatus for vaporizing insecticidal solutions and the like**

(30) Priority: 06.04.1995 IT BO950154
(71) Applicant: FALP S.r.l., I-40060 Passo Segni Baricella, Bologna (IT)
(72) Inventor: Falchieri, Roberto, 40060 Baricella (Bologna) (IT)
(74) Representative: Modiano, Guido, Dr.-Ing.

(57) **Abstract**

The apparatus vaporizes insecticidal solutions and the like placed in a container (3) from which a portion of an element made of absorbing material, or wick (4), protrudes. The container (3) has a neck (13) that is adapted to fit inside a sleeve (9) that is formed axially by a casing (1) that forms the seat for heating means that are adapted to heat the wick (4). The neck (13) is provided, in an upward region, with threaded means (14) that are adapted for screwing with corresponding female screw (15) means formed on the inside surface of the sleeve (9) and, in a base portion, with a collar (17) that is adapted to engage in a snap-together manner, during the screwing action, shoulder means (18) that are formed by the sleeve (9), so as to axially rigidly couple the container (3) to the casing (1).

## Description

The present invention relates to an apparatus for vaporizing insecticidal solutions and the like.

Heating devices are currently known which cause the evaporation of vaporizable solutions that contain active insecticidal or deodorant substances. These vaporizable solutions are placed in a container in which an element made of absorbing material, or wick, is inserted; said wick dips into the solution. The absorbing element is commonly made of high-porosity materials, such as for example paper, felt, and the like, or of low-porosity materials, such as ceramic and the like.

In order to achieve evaporation of the solution, a portion of the absorbing element that protrudes from the container of the vaporizable solution is placed close to the heating means of the apparatus, which is generally powered electrically.

A conventional apparatus includes a casing that is adapted to be associated with said container and forms the seat for the heating means that are adapted to heat the wick.

In the above-mentioned devices there is the problem of avoiding accidental contact, for example of children, with the wick soaked in the active substance.

The aim of the present invention is to solve the above problem, providing an apparatus for vaporizing insecticidal solutions and the like that allows safe use, particularly allowing to avoid unwanted contacts with the wick.

Within the scope of this aim, an object of the present invention is to provide an apparatus that is simple in concept, safely reliable in operation, and versatile in use.

This aim and this object are both achieved, according to the invention, by the present apparatus for vaporizing insecticidal solutions and the like which comprises a container from which a portion of an element made of absorbing material, or wick, protrudes, and a casing that is adapted to be associated with said container and forms the seat for heating means that are adapted to heat said wick, characterized in that said container has a neck that is adapted to fit inside a sleeve that is formed axially by said casing and is provided, in an upward region, with threaded means that are adapted for screwing with corresponding female screw means formed on the inside surface of said sleeve and, in a base portion, with a collar that is adapted to engage in a snap-together manner, during said screwing action, shoulder means that are formed by said sleeve, so as to axially rigidly couple said container to said casing.

The details of the invention will become apparent from the detailed description of a preferred embodiment of the apparatus for vaporizing insecticidal solutions and the like, illustrated only by way of non-limitative example in the accompanying drawings, wherein:

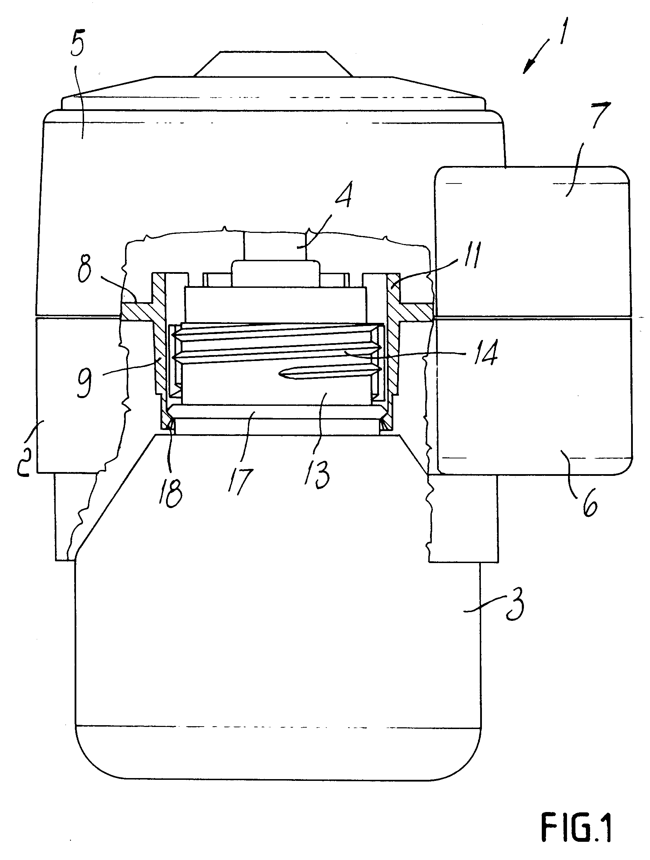

figure 1 is a partially cutout and sectional side view of the apparatus according to the invention;

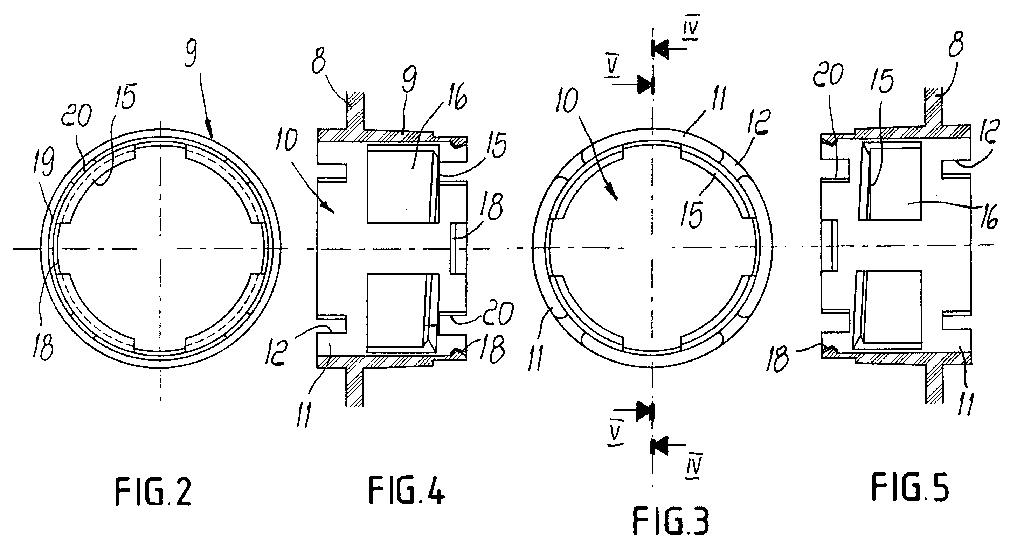

figure 2 is a bottom plan view of a portion of said casing that forms said female screw means;
figure 3 is a corresponding top plan view thereof;
figures 4 and 5 are axial sectional views of said portion of the casing of the apparatus, taken respectively along the planes IV-IV and V-V of figure 3.

With particular reference to said figures, the reference numeral 1 generally designates the casing of the apparatus according to the invention, which is preferably made of molded plastics. The casing 1 has a substantially cylindrical body 2 that is internally hollow and open in a downward region for coupling to a container 3 for the vaporizable solution that is constituted in practice by a small bottle made for example of transparent plastics or glass; a portion of an element made of absorbing material, or wick 4, which dips into the solution protrudes axially from said container 3.

A lid 5 is adapted to be associated with the body 2 and is correspondingly cylindrical with a slight taper. The lid 5 has a series of protruding pins, not shown in the drawing, inside it; said pins are adapted for snap-together coupling with a corresponding series of tubular couplings that protrude in an upward region from the body 2.

The body 2 and the lid 5 of the casing 1 laterally form, respectively, the mutually complementary lower portion 6 and upper portion 7 of a box-like bracket that provides rotary coupling to an electric plug. The portions 6 and 7 of said bracket are in fact frontally provided with respective halves of a circular opening that is meant to engage an annular groove formed by the electric plug.

The casing 1 forms, in a conventional manner, the seat for appropriate heating means that are adapted to heat said wick 4 and are not shown in the drawing. Of course, these heating means, for example appropriate resistors, are powered electrically by means of said plug.

The body 2 has an upper surface 8 that axially forms a sleeve 9 that is directed downwards and forms a central opening 10 of said body 2; the sleeve 9 is continued axially by a ring 11 that protrudes from the upper surface 8 of the body 2, at the margin of the central opening 10. The ring 11 is interrupted by a series of uniformly distributed slots 12; in practice, the ring 11 is constituted by four annular sectors that are separated by corresponding slots 12.

The neck 13 of the container 3, which is cylindrical, enters through the sleeve 9. Said neck 13 has, on its outer surface, in an upper portion, a helical protrusion 14 that has a triangular cross-section and is adapted to form the thread for screwing the container 3 to the body 2.

The thread 14 is adapted to engage, in a screwing position, a female screw that is constituted by a series of raised portions 15 that have a triangular cross-section and protrude from corresponding plates 16 that are formed on the inside surface of the sleeve 9; in practice, said female screw is constituted by four raised portions 15 that are arranged along a helical path and are uniformly spaced.

The neck 13 also has, at the base, on its outer surface, a collar 17 that has a triangular cross-section and has a wider diameter than the thread 14. The collar 17 is adapted to abut, in said screwing position, against an annular shoulder 18 that is formed, on the inside surface of the sleeve 9, by a series of toothed sectors that are uniformly spaced; said sectors 18, which have a triangular profile, are practically arranged in alternation with the raised portions 15.

More specifically, the sectors 18 of the shoulder are formed proximate to the margin of a lower portion 19 of the sleeve 9 that has a reduced thickness. Said portion 19 also has a series of slots 20 that are uniformly distributed and are, in practice, arranged in alternation with said sectors 18 of the shoulder.

To use the device, the container 3 of the vaporizable solution is screwed onto the body 2 of the casing 1. This screwing is achieved, as mentioned, by the engagement of the thread 14 formed on the neck 13 of the container 3 with the raised portions 15 that form a corresponding female screw on the sleeve 9 of the body 2.

At the end of the screwing stroke, an additional rotation imparted to the container 3 allows the collar 17 of the neck 13 to pass, with a snap action, beyond the shoulder, formed the by sectors 18, that is provided on the lower portion 19 of the sleeve 9; said lower portion 19 in fact flexes elastically outwards, in contact with the collar 17, by virtue of the flexibility provided by the reduced thickness and by the presence of the slots 20.

In this configuration, the container 3 is associated, so that it can rotate freely, with the body 2 of the casing 1 of the apparatus and is axially rigidly coupled thereto. In practice, it is not possible to disengage the container 3 from the body 2 by unscrewing it.

In order to disengage the container 3, it is necessary to apply a pulling action to the container 3 in the direction of extraction from the casing 1, thereby causing the collar 17 to pass beyond the retainer constituted by the shoulder 18. This is allowed by the elastic flexing of the lower portion 19 of the sleeve 9 that is provided with the toothed sectors that form said shoulder 18. It is then possible to unscrew the thread 14 from the female screw 15.

The described apparatus allows to install the container 3 of the solution on the casing very easily and quickly, by screwing, but prevents subsequent extraction thereof performed by simply unscrewing.

This of course allows to avoid unwanted contacts, for example of children, with the wick soaked with the solution. The apparatus can therefore be used in absolute safety for the vaporization of insecticidal solutions and the like.

Another advantage provided by the apparatus according to the invention is constituted by the fact that the described solution entails a limited number of parts, conveniently produced by molding plastic materials and the like. In particular, the container of the solution is formed monolithically and is adapted to be closed by the element that forms the wick.

In the practical embodiment of the invention, the materials employed, as well as the shapes and the dimensions, may be any according to the requirements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. Apparatus for vaporizing insecticidal solutions and the like, of the type that comprises a container from which a portion of an element made of absorbing material, or wick, protrudes, and a casing that is adapted to be associated with said container and forms the seat for heating means that are adapted to heat said wick, characterized in that said container has a neck that is adapted to fit inside a sleeve that is formed axially by said casing and is provided, in an upward region, with threaded means that are adapted for screwing with corresponding female screw means formed on the inside surface of said sleeve and, in a base portion, with a collar that is adapted to engage in a snap-together manner, during said screwing action, shoulder means that are formed by said sleeve, so as to axially rigidly couple said container to said casing.

2. Apparatus according to claim 1, characterized in that said shoulder means are provided proximate to the margin of a lower portion of said sleeve that is thinner and has a series of uniformly distributed slots.

3. Apparatus according to claim 1, characterized in that said shoulder means are formed by a series of toothed sectors that have a triangular profile, are uniformly spaced, and are arranged in alternation with a series of slots formed on the margin of said sleeve.

4. Apparatus according to claim 1, characterized in that said female screw means are constituted by a series of raised portions that have a triangular cross-section, protrude from the inside surface of said sleeve, and are arranged so that they are uniformly spaced along a helical path.

5. Apparatus according to claim 1, characterized in that said collar has a triangular cross-section and a wider diameter than said threaded means.
